# EUROPEAN PATENT SPECIFICATION

(11) **EP 0 497 041 B1**
(45) Date of publication and mention of the grant of the patent: **08.01.1997**
(21) Application number: 91310459.2
(22) Date of filing: 13.11.1991
(51) Int. Cl.: A61M 5/172

(54) **Automated infusion pump with replaceable memory cartridges**
Automatische Infusionspumpe mit austauschbarem Datenmodul
Pompe de perfusion automatique avec module de mémoire remplaçable

(30) Priority: 31.01.1991 US 648600
(43) Date of publication of application: 05.08.1992
(73) Proprietor: BAXTER INTERNATIONAL INC., Deerfield, IL 60015 (US)
(72) Inventor: Samiotes, Nicholas, Westwood, Massachusetts (US); Johanson, Donald, Wayland, Massachusetts (US); Tamura, Paul, Seattle, Washington (US)
(74) Representative: JENSEN & SON

(56) References cited:
- EP-A- 0 002 775
- EP-A- 0 188 288
- CH-A- 665 955

## Description

This invention pertains to a microprocessor controlled infusion pump for delivering drugs to a patient, and more particularly to an infusion pump with a memory cartridge which defines one of a variety of configurations for the pump, said cartridges being replaceable to allow the pump to be used in multiple, different environments.

Infusion pumps are used in the field of medicine to administer drugs to patients over an extended time period particularly durations longer than can be managed easily by direct injection. As availability of drugs, therapeutic techniques, and technological capability have improved, the demand for sophistication in drug delivery has increased. In many instances, this added sophistication results in more complicated device operation. Achieving this sophistication in drug delivery capability, and maintaining ease of use has been a major challenge for infusion pump manufacturers.

One reason that devices have become complicated is the breadth of drug dosing methods used. For example, such different modes of operation as Patient Controlled Analgesia (PCA) and physician interactive dosing for operating room infusions must somehow be accommodated. Other current therapy modes in use include continuous infusion, bolus plus continuous infusion, clinician interactive dosing; PCA with continuous infusion, time/dose, and multiple automatic time/dose.

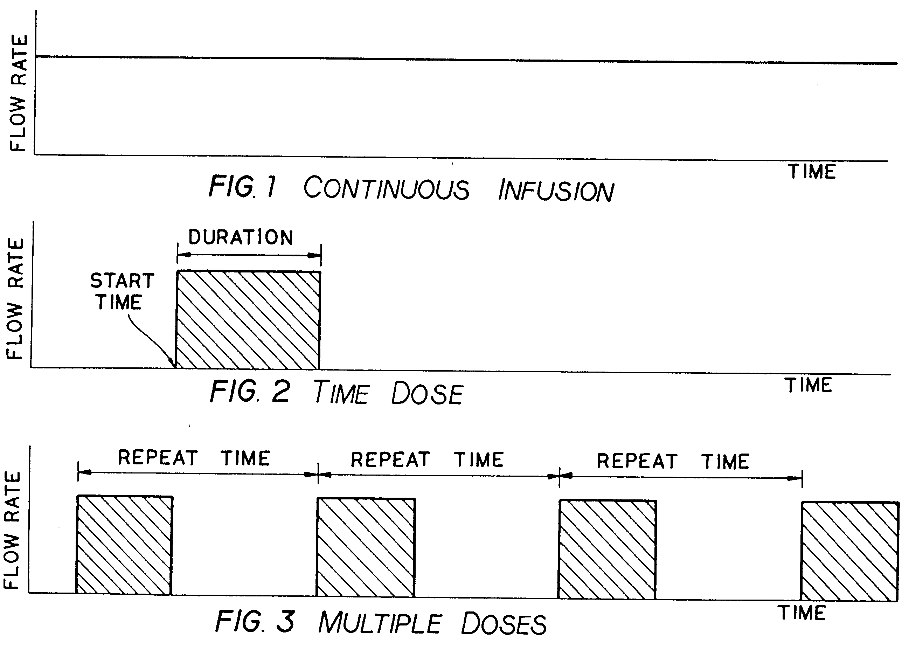

Continuous infusion is the most traditional method of drug delivery. Figure 1 shows a graph of a constant drug flow vs. time. The infusion rate may be changed, if necessary, but whatever rate is selected will continue indefinitely. In this type of infusion, the user only selects the rate in m1/hr or drops/minute.

Time/dose therapy is shown in figure 2. This therapy is accomplished with a number of different devices for many different drugs. One device, a syringe infusion pump described in U.S. Patent 4,544,369, has no rate input at all. The selection of syringe size and dose volume define the duration of infusion and, thus, the flow rate. Other devices have flow rate and dose volume as inputs. Still others require dose volume and duration of infusion as inputs. Intermittent medications such as antibiotics and H₂ agonists are often administered using these devices. Swiss Patent CH 665955 discloses an injecting device for a drug comprising a drug reservoir within an interchangeable cartridge, a dedicated infusion pump and an electronic circuit controlling the pump operation. The dose and injection-timing information is entered into the device via buttons. To avoid errors in the dosage, information can only be entered if a card having a microprocessor is inserted into the device. The cards permit the selection from possible ranges of information according to the drug to be administered. A disadvantage of such a device is that the pump must be changed for different drug infusions. The present invention overcomes this by providing a pump which can be programmed to mimic a selected one of a range of pumps.

Figure 3 shows a multiple dose scheme. This method is similar to time/dose, but also includes a repeat time as a user defined parameter. The pump automatically starts each dose at predetermined time intervals.

Figure 4 shows patient controlled analgesia (PCA) therapy. The clinician typically selects a dose size, and a minimum delay or lockout period. The patient may request a drug dose using a bedside request button connected to the pump. The pump administers a dose only if it has been requested during the minimum time period after the administration of a previous dose.

Often, a continuous infusion is superimposed over PCA therapy as shown in Figure 5. The clinician must select a continuous infusion rate along with the dose size and minimum delay period. PCA therapy may also include additional clinician selectable features. These include a loading dose at the start of or during therapy and secondary prescription limits (e.g. hourly limit).

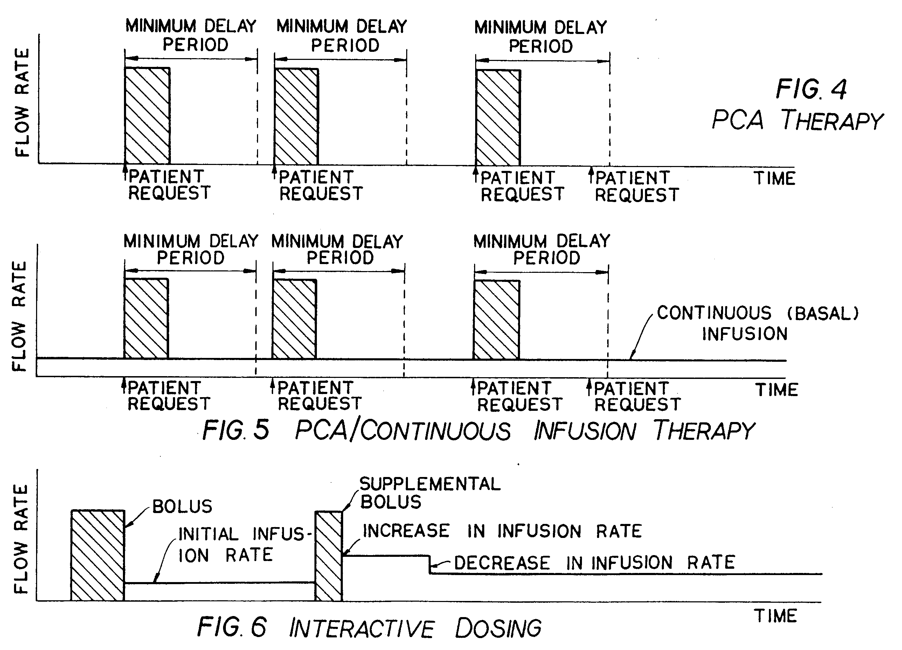

Interactive dosing is commonly done in the operating room (OR) or the intensive care unit (ICU). Figure 6 shows a typical course of therapy. The clinician selects appropriate rates, boluses, and changes based on patient response or therapeutic goal. Aspects of interactive dosing are common to all infusion schemes because the actual dosing requirements change from patient to patient and from time to time. Nevertheless, certain circumstances require rapid interactive dosing not necessarily available or even desirable on all devices. An example of a device particularly well suited for this type of interactive dosing is made by C.R. Bard and is described in U.S. Patent number 4,943,279.

Of particular importance to the foregoing discussion is the wide variety of user selectable primary dosing inputs. The range of possible user inputs includes, but is not limited to, infusion rate, infusion duration, dose volume, lockout period, repeat period, bolus size, and bolus rate. Secondary inputs may include patient weight, patient sex, syringe size, container volume, security codes, drug units (e.g. milligrams), drug concentration, or even pharmacokinetic parameters.

In addition, not only are the clinician inputs numerous and variable, but the infusion pump's outputs are also extensive. History and status information unique to each therapy must be presented to the user in a clear manner.

The totality of inputs and outputs is termed the user interface. This user interface often has one most desirable features for a particular therapeutic modality.

Because of the large number of therapeutic modalities and the extensive number of possible required inputs and outputs, the challenge to manufacturers has been great. Users desire most a user interface with only a particular set of inputs and outputs pertinent to a particular therapy. On the other hand, great versatility is desired by both the manufacturers and the users. Users desire versatility so their pumps can perform many therapies. Manufacturers have the same desire because the economies of scale can be more easily realised.

The infusion industry has collectively responded to these needs in a number of ways. First, compromises in features are often accepted in the interest of having versatile, general or multi-purpose pumps. For example, users may sometimes select doses based on body weight and drug units, but often this may not be current practice for some drugs. Therefore when necessary, users perform conversion calculations so that rates may be selected in m1/hr. These calculations are time consuming and are potential error sources. Another example is that continuous infusion modes may be used for time/dose infusions. The user must either time the infusion or rely on "end of container" alarms to appropriately terminate delivery.

An alternative to general purpose pumps has been devices designed specifically for only one drug or therapy. This has proven desirable for some widespread therapies. Users value the simple operation of these devices since they include only those features needed for a particular therapy. The disadvantages of these devices is their lack of versatility. They usually cannot perform widely differing therapies, nor can they accommodate therapy advance over time.

Swiss Patent No. 665 955 discloses a dosing apparatus which attempts to solve these problems by providing three separate pump/control cartridge modules, each designed to correspond to different categories of pharmaceutical products. Each pump covers a certain range and the operator has to select the most appropriate pump for his purpose. The operating parameters of the selected pump are selected by the operator from the presented range.

The present invention seeks to overcome the above-mentioned disadvantages of the prior art by providing an infusion pump system with variable user interface characteristics in which a single infusion pump is used to mimic a plurality of dedicated pumps.

A further objective is to provide an infusion pump system which may be operated as a pump having particular user interface characteristics by using a replaceable memory module.

Yet another objective is to provide a pump system with user interface means thereon for adjusting the operation of the system in accordance with the needs and requirements of a particular patient.

Other objectives and advantages of the invention shall become apparent from the following description.

According to the present invention there is provided a universal infusion pump assembly for configuring a single infusion pump to mimic the operation of one of a plurality of dedicated infusion pumps, said assembly comprising:
a housing means for positioning adjacent to a patient for delivering a fluid to said patient;
reservoir means coupled to said housing for holding said fluid;
delivery means for delivering said fluid to said patient from said reservoir;
input means mounted on said housing for inputting patient specific information;
microprocessor means for controlling said delivery means; and a module selected from a set of modules, characterised in that the assembly comprises a single infusion pump, said module being a memory module selected from a set of memory modules, each module of the set being cooperable with said single infusion pump, the selected memory module containing a program describing a specific delivery profile characteristic of a respective dedicated pump, said microprocessor means being coupled to said memory module, said microprocessor means reading said program and said patient specific information to create control parameters indicative of a specific patient and operating said delivery means to emulate the respective dedicated pump.

The memory module, which may comprise a cartridge, is used to configure the infusion pump system, and particularly the control means, to a particular user interface and to particular functional characteristics. The control means further includes keypads, rotary knobs, or other means of inputting specific dosing or patient information to the microprocessor. The control means further still includes display, alarm, or other means for communicating with the user. The control means also provides operating and monitoring means indicative of the internal functions of the infusion pump.
Figure 1-6 show time charts for various kinds of infusion that can be performed with an infusion pump constructed in accordance with this invention;

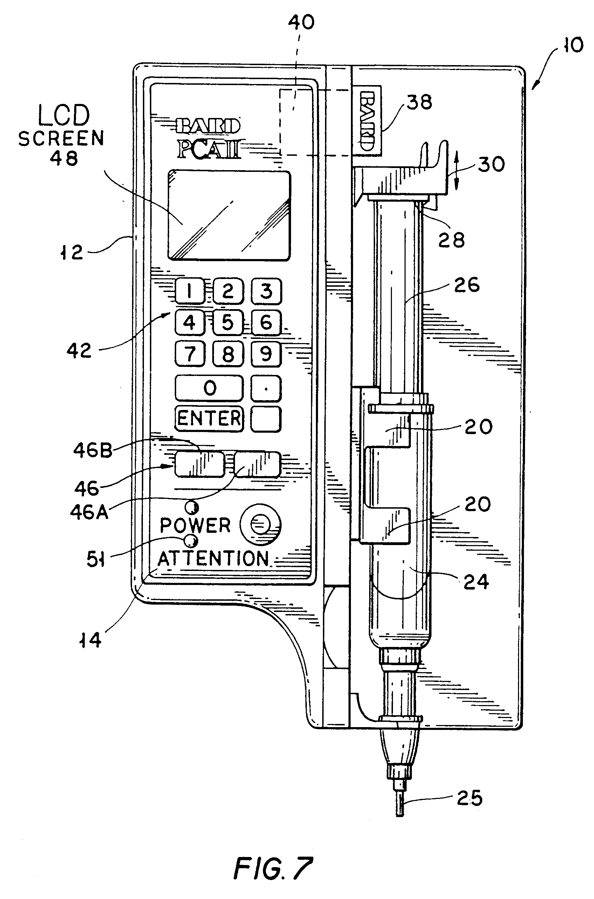

Figure 7 shows an elevational view of an infusion pump constructed in accordance with this invention;

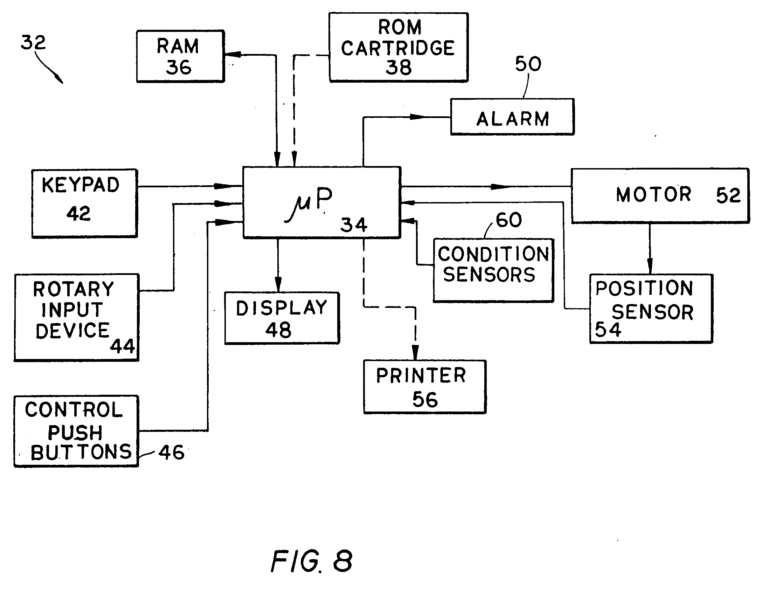

Figure 8 shows a schematic diagram for the control of the infusion pump of Figure 7; and

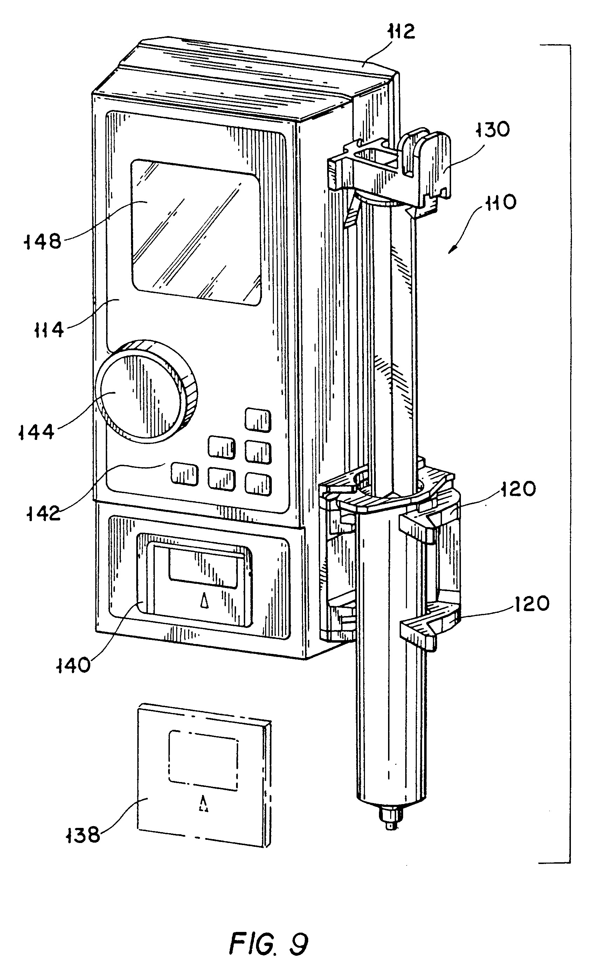

Figure 9 shows an elevational view of an alternate embodiment of the infusion pump.

As shown in the Figures, and more particularly in Figure 7 an infusion pump 10 may include a housing 12 with a front 14 used to hold various control and display means used to set up and operate the pump as described more fully below. The housing 12 may include mounting means (not shown) for securing the pump to an IV pole or another stationary frame.

On one side of the housing the pump is provided with a bracket 20, for engaging and holding the barrel 24 of a syringe. The bracket 20, may include sensors for sensing the diameter of syringe barrel 24. The syringe also includes a plunger 26 reciprocated in the barrel 24. The plunger 26 is terminated by a flange 28 which is captured by a plunger activating assembly 30. Assembly 30 is coupled to and driven by a motor (shown in Figure 8) up or down to permit the plunger 26 to be inserted into or out of syringe barrel 24. Thus, in Figure 7 the downward movement of assembly 30 causes the plunger 26 to eject the contents of the syringe barrel 24 either directly into the blood stream of a patient through a catheter device or into an IV through line 25. Therefore, the delivery of the drug from syringe barrel 24 to the patient is controllable through the vertical downwardly movement of the assembly 30 as well as the size of the syringe.

Inside the housing 12, there is the motor mentioned above for driving assembly 30, and a control system 32 shown in more detail in Figure 8. The control system 32 includes a microprocessor 34, which is coupled to a RAM 36 as well as several input and output devices. RAM 36 is the random access memory for the microprocessor and holds appropriate memory for the pump. Importantly, the system 32 also includes replaceable ROM cartridge 38. As shown in Figure 7, the housing 12 has a slot 40 accessible from the outside for holding ROM cartridge 38 so that the ROM cartridge 38 is readily removable and replaceable with another cartridge. Instructions and patient specific data are provided to microprocessor 34 from a standard keypad 42 and some additional control push-buttons 46. All these input devices are mounted on the face 14 of housing 12 as shown in Figure 7. In addition the system is also provided with a screen such as an LCD-type screen 48, and alarms, including attention light 51 used to indicate an abnormal pump operation. The size of barrel 24 is determined from condition sensors 60 which may be mounted in brackets 20, 22. Microprocessor 34 generates control signals to a mechanical which are used to drive motor 52 for activating the plunger. The position of motor 52 is sensed by a motor sensor 54 and sent back to the microprocessor for verification. Optionally, a printer interface may also be provided for printing data from the microprocessor 34 to an external printer 56.

The general operation of the infusion pump shall now be described. Initially, a ROM cartridge 38 corresponding to a specific delivery profile, such as one of the profiles shown in Figures 1-6, is loaded into slot 40. In other words a ROM 38 is provided for each specific user interface, thereby defining the characteristics of a dedicated pump. The microprocessor 34 reads this information from the cartridge 38. Next, the microprocessor provides operational instructions on LCD display 48 and, if necessary, requests patient-specific information. In other words, display 48 is used to request dosing inputs such as flow rate, dose size, patient weight, drug concentration, etc., as required. The display 48 may also be used to identify the cartridge inserted in slot 40, and other initial information such as the size of the barrel as determined by the sensors 60, thereby providing a further means to ensure that the pump is operated properly and that the patient will receive the correct drug dose. The operator of the pump enters the requested information through the key pad 42. The remaining control push buttons 46 may be dedicated to other functions such as a review function (46A) during which the information provided to the microprocessor is reviewed prior to the operation of the pump, or to start or stop the pump, by activating push button 46B.

Once the requested information is entered, the microprocessor 34 adjusts the operational parameters for the pump to meet the requirements of the specific patient, and on command, for example, from push button 46B starts the infusion. The microprocessor monitors the operation of the pump on a continuous basis, and in case of a malfunction, it activates alarm light 51 and/or any other alarms 50.

To summarize, the pump 10 may be used for infusion using the characteristic/parameters corresponding to a specific user interface defined by cartridge 38 to act as a particular type of, or dedicated pump. The operation parameters and user interface of the pump, as well as the procedure for administering a particular drug are all stored in a cartridge 38. Patient specific information and other information, are provided to the microprocessor during an initialization phase via the input means provided on face 14. As desired, cartridge 38 may be changed to a different user interface to implement another pump having different operational parameters, for administering a different drug or therapy. In this manner a single type of infusion pump is used to emulate a large number of pumps. If technical advances and/or new regulations require a new infusion profile, the pump is easily reconfigured by providing an appropriate cartridge 38. Furthermore, the cartridges are easily installed in the field by personnel with no programming knowledge or capability.

Figure 9 shows an alternate embodiment 110 of the infusion pump. Pump 110 includes housing 112 with bracket 120, disposed on one side with a corresponding plunger activating assembly 130. Housing 112 also has a face 114 with data entry and control push buttons 142, a rotary switch 144 and an LCD screen 148. Disposed below the key 142, there is a slot 140 for accepting a ROM cartridge 138. The operation of this embodiment is identical to the operation of the pump illustrated in types 7 and 8.

The infusion pump described herein has a number of benefits and advantages. A single pump may be used to serve multiple needs of a user in a single facility. The operation of the device is simplified since the instructions for setting up the pump for a particular operation are presented to the user one step at a time on the screen.

Existing applications, for example, to conform to changes in drug concentrations, acceptable usage, etc. may be easily updated with out changing the pump. The pump can be easily configured for future applications.

Moreover, because each cartridge dedicates a pump to a specific pump configuration for each application, the pump is only as complicated as needed to perform those applications.

Software upgrades are easily implemented, thereby ensuring greatly the pump's useful life.

The pump can be easily configured for special applications, thereby reducing development costs considerably.

The pump can be easily configured to accommodate new drugs and techniques.

Finally, a user is free to buy only the ROM cartridges for his specific application, with the option of buying more as required, or as they because available. Although the memory modules are described as ROM cartridges it will be appreciated that other forms of date storage such as discs or CD Roms may be used.

Obviously numerous modifications can be made to this invention without departing from its scope as defined in the appended claim

## Claims

1. A universal infusion pump assembly for configuring a single infusion pump to mimic the operation of one of a plurality of dedicated infusion pumps, said assembly comprising:
a housing means (12) for positioning adjacent to a patient for delivering a fluid to said patient;
reservoir means (24) coupled to said housing for holding said fluid;
delivery means (22, 25, 26, 28, 52) for delivering said fluid to said patient from said reservoir;
input means (42) for inputting patient specific information;
microprocessor means (34) for controlling said delivery means, and a module selected from a set of modules, characterised in that the assembly comprises a single infusion pump, said module being a memory module (38) selected from a set of memory modules, each module of the set being cooperable with said single infusion pump, the selected memory module containing a program describing a specific delivery profile characteristic of a respective dedicated pump, said microprocessor means (34) being coupled to said memory module (38), said microprocessor means reading said program and said patient specific information to create control parameters indicative of a specific patient and operating said delivery means (25) to emulate the respective dedicated pump.

2. A pump assembly according to claim 1, further comprising display means (48) mounted on said housing means for displaying output data from said microprocessor.

3. A pump assembly according to claim 2, wherein said display means (48) includes a display panel for displaying operation instructions.

4. A pump assembly according to claim 2 or 3, wherein said display means (48) includes an alarm indicator for indicating an abnormal operating condition.

5. A pump assembly according to any one of claims 1 to 4, further comprising manual control means (46) mounted on said housing means.

6. A pump assembly according to claim 5 wherein said manual control means (46) includes manual keys disposed on said housing means.

7. A pump assembly according to claim 5 wherein said manual control means (46) includes a keypad having several keys selected by an operator.

8. A method of operating a universal infusion pump assembly for configuring a single infusion pump to mimic the operation of one of a plurality of dedicated infusion pumps, comprising the steps of:
a. providing an infusion pump system having a reservoir (24) with a drug, delivery system means (25) for delivering said drug to a patient including drug pump means (10), delivery control means (32) including a microprocessor (34) for operating said delivery means, a set of modules (38) and input means (42) for generating patient specific information for said patient, characterised in that the assembly comprises a single infusion pump, that each module (38) of the set is cooperable with said single infusion pump, and that the set of modules is a set of memory modules, each module containing a program describing a specific delivery profile characteristic of a respective dedicated pump; the method further comprising the steps of:
b. selecting a specific memory module (38);
c. coupling said specific memory module (38) to said microprocessor (34), wherein said microprocessor reads said program; and
d. generating patient specific information to said microprocessor through said input means whereby said microprocessor operates said delivery system (25) to emulate the respective dedicated pump.

## Patentansprüche

1. Universelles Infusionspumpen-Aggregat zum Konfigurieren einer einzelnen Infusionspumpe, um die Betriebsweise einer Pumpe aus einer Vielzahl von bestimmten Infusionspumpen nachzuahmen, welches Aggregat umfaßt:
eine Gehäuseeinheit (12) zur Positionierung an einem Patienten zur Abgabe einer Flüssigkeit an den Patienten;
eine Reservoireinheit (24) , die mit dem Gehäuse zum Speichern der Flüssigkeit gekoppelt ist;
eine Abgabeeinheit (22, 25, 26, 28, 52) zur Abgabe der Flüssigkeit an den Patienten aus dem Reservoir;
eine Eingabeeinheit (42), um patientenspezifische Informationen einzugeben;
eine Mikroprozessoreinheit (34) zur Steuerung der Abgabeeinheit und ein Modul, das aus einem Satz von Modulen ausgewählt ist, dadurch gekennzeichnet, daß das Aggregat eine einzelne Infusionspumpe enthält, daß das Modul ein Speichermodul (38) ist, das aus einem Satz von Speichermodulen ausgewählt ist, und daß jedes Modul (38) des Satzes zusammenarbeitsfähig mit der einzelnen Infusionspumpe ist, wobei das ausgewählte Speichermodul ein Programm enthält, das eine spezifische Abgabeprofil-Kennlinie einer jeweiligen bestimmten Pumpe beschreibt, wobei die Mikroprozessoreinheit (34) mit dem Speichermodul (38) gekoppelt ist, die Mikroprozessoreinheit das Programm und die patientenspezifischen Informationen ausliest, um Steuerparameter zu erzeugen, die für einen speziellen Patienten repräsentativ sind, und die Abgabeeinheit (25) betätigt, um eine Emulation der jeweiligen bestimmten Pumpe durchzuführen.

2. Pumpenaggregat nach Anspruch 1, weiterhin umfassend eine Anzeigeeinheit (48), die an der Gehäuseeinheit befestigt ist, um Ausgangsdaten von dem Mikroprozessor anzuzeigen.

3. Pumpenaggregat nach Anspruch 2, wobei die Anzeigeeinheit (48) eine Anzeigetafel zur Anzeige von Betätigungsinstruktionen umfaßt.

4. Pumpenaggregat nach Anspruch 2 oder 3, wobei die Anzeigeeinheit (48) eine Alarmanzeige zum Anzeigen eines abnormalen Betriebszustandes aufweist.

5. Pumpenaggregat nach einem der Ansprüche 1 bis 4, weiterhin umfassend manuelle Steuereinrichtungen (46), die an der Gehäuseinheit montiert sind.

6. Pumpenaggregat nach Anspruch 5, wobei die manuellen Steuereinrichtungen (46) manuelle Tasten umfassen, die an der Gehäuseinheit angeordnet sind.

7. Pumpenaggregat nach Anspruch 5, wobei die manuellen Steuereinrichtungen (46) eine Tastatur umfassen, die mehrere, von der Bedienungsperson selektierbare Tasten aufweist.

8. Verfahren zum Betrieb eines universellen Infusionspumpenaggregates zum Konfigurieren einer einzelnen Infusionspumpe, um den Betrieb einer Pumpe aus einer Vielzahl von bestimmten Infusionspumpen nachzuahmen, mit folgenden Verfahrensschritten:
a) Schaffen eines Infusionspumpensystems, das ein Reservoir (24) mit einem Medikament, eine Abgabesystemeinheit (25) zur Abgabe des Medikaments an einen Patienten, die eine Medikamentenpumpeneinheit (10) umfaßt, Abgabesteuereinrichtungen (32) umfassend eines Mikroprozessor (34) zur Betätigung der Abgabeeinrichtung, einen Satz von Modulen (38) und einer Eingabeeinheit (42) zur Erzeugung von patientenspezifischer Information für den Patienten umfaßt, dadurch gekennzeichnet, daß das Aggregat eine einzelne Infusionspumpe umfaßt, daß jedes Modul (38) des Satzes zusammenarbeitsfähig mit der einzelnen Infusionspumpe ist und daß der Satz von Modulen ein Satz von Speichermodulen ist, wobei jedes Modul ein Programm enthält, das eine spezielle Abgabeprofilcharakteristik einer bestimmten ausgewählten Pumpe beschreibt; wobei das Verfahren weiterhin die Verfahrensschritte enthält:
b) Auswählen eines bestimmten Speichermoduls (38);
c) Koppeln des speziellen Speichermoduls (38) mit dem Mikroprozessor (34), wobei der Mikroprozessor das Programm ausliest; und
d) Übergabe von patientenspezifischen Informationen an den Mikroprozessor über die Eingabeeinrichtung, wodurch der Mikroprozessor das Abgabesystem (25) so tätigt, daß die jeweils ausgewählte Pumpe emuliert wird.

## Revendications

1. Ensemble formant pompe à perfusion universelle pour configurer une unique pompe à perfusion pour simuler le fonctionnement de l'une d'une pluralité de pompes à perfusion dédiées, ledit ensemble comportant :
• un moyen formant boîtier (12) pour le positionnement près d'un patient pour administrer un fluide audit patient ;
• des moyens formant réservoir (24) couplés audit boîtier pour contenir ledit fluide ;
• des moyens d'administration (22, 25, 26, 28, 52) pour administrer ledit fluide audit patient à partir dudit réservoir ;
• des moyens d'entrée (42) pour entrer une information spécifique au patient ;
• des moyens formant microprocesseur (34) pour commander lesdits moyens d'administration ; et un module (38) choisi parmi un ensemble de modules caractérisé en ce que l'ensemble comporte une unique pompe à perfusion, le module étant un module de mémoire choisi parmi un ensemble de modules de mémoire et en ce que chaque module (38) de l'ensemble peut coopérer avec ladite unique pompe à perfusion, le module de mémoire choisi contenant un programme décrivant une caractéristique de profil spécifique d'administration d'une pompe dédiée respective, lesdits moyens formant microprocesseur (34) étant couplés audit module de mémoire (38), lesdits moyens formant microprocesseur lisant ledit programme et ladite information spécifique au patient pour créer les paramètres de commande indicatifs d'un patient spécifique et faisant fonctionner lesdits moyens d'administration (25) pour constituer une émulation de la pompe dédiée respective.

2. Ensemble formant pompe selon la revendication 1, comportant en outre des moyens de visualisation (48) montés sur lesdits moyens formant boîtier pour visualiser les données émises par ledit microprocesseur.

3. Ensemble formant pompe selon la revendication 2, dans lequel lesdits moyens de visualisation (48) incluent un panneau de visualisation pour visualiser des instructions de fonctionnement.

4. Ensemble formant pompe selon la revendication 2 ou 3, dans lequel lesdits moyens de visualisation (48) incluent un indicateur d'alarme pour indiquer une condition de fonctionnement anormale.

5. Ensemble formant pompe selon l'une quelconque des revendications 1 à 4, comportant en outre des moyens de commande manuelle (46) montés sur lesdits moyens formant boîtier.

6. Ensemble formant pompe selon la revendication 5, dans lequel lesdits moyens de commande manuelle (46) incluent des touches manuelles disposées sur lesdits moyens formant boîtier.

7. Ensemble formant pompe selon la revendication 5, dans lequel lesdits moyens de commande manuelle (46) incluent un clavier comportant plusieurs touches choisies par un opérateur.

8. Procédé de fonctionnement d'un ensemble formant pompe à perfusion universelle pour configurer une unique pompe à perfusion pour simuler le fonctionnement de l'une d'une pluralité de pompes à perfusion dédiées, comportant les étapes consistant à :
a. disposer d'un système de pompe à perfusion comportant un réservoir (24) rempli d'un médicament, des moyens (25) formant système d'administration pour administrer ledit médicament à un patient, incluant des moyens (10) de pompage du médicament, des moyens (32) de commande d'administration incluant un microprocesseur (34) pour faire fonctionner lesdits moyens d'administration, un ensemble de modules (38) et des moyens d'entrée (42) pour générer une information spécifique au patient pour ledit patient, caractérisé en ce que l'ensemble comprend une pompe de perfusion unique, en ce que chaque module de l'ensemble est adapté à coopérer avec la pompe à perfusion unique et en ce que l'ensemble de modules est un ensemble de modules de mémoire, chaque module contenant un programme décrivant une caractéristique du profil d'administration spécifique d'une pompe dédiée respective ; le procédé comportant en outre les étapes consistant à :
b. choisir un module de mémoire spécifique (38) ;
c. coupler, audit microprocesseur (34) ledit module de mémoire spécifique (38) dans lequel ledit microprocesseur lit ledit programme ; et
d. fournir audit microprocesseur une information spécifique au patient par l'intermédiaire desdits moyens d'entrée, de sorte que ledit microprocesseur actionne ledit système d'administration (25) pour constituer une émulation de la pompe dédiée respective.
